# EUROPEAN PATENT SPECIFICATION

(11) **EP 1 817 320 B1**
(45) Date of publication and mention of the grant of the patent: **30.06.2010**
(21) Application number: 05813632.6
(22) Date of filing: 18.11.2005
(51) Int. Cl.: C07H 13/04, C07H 13/10, C07H 1/00

(54) **FLUORIDATION PROCESS FOR THE SYNTHESIS OF 2-[18F] FLUORO-2-DEOXY-D-GLUCOSE**
FLUORISIERUNGSVERFAHREN ZUR SYNTHESE VON 2-[18F] FLUORO-2-DEOXY-D-GLUKOSE
PROCÉDÉ DE FLUORURATION POUR LA SYNTHÉSE DU 2-[187]-FLUORO-2-DÉOXY-D-GLUCOSE

(30) Priority: 19.11.2004 GB 0425501
(43) Date of publication of application: 15.08.2007
(73) Proprietor: GE Healthcare Limited, Little Chalfont Buckinghamshire HP7 9NA (GB)
(72) Inventor: OSBORN, Nigel John, GE Healthcare Limited, Buckinghamshire HP7 9LL (GB); GRIGG, Julian, GE Healthcare Limited, Buckinghamshire HP7 9LL (GB); PETTITT, Roger Paul, GE Healthcare Limited, Buckinghamshire HP7 9LL (GB); WILSON, Anthony, GE Healthcare Limited, Amersham, Buckinghamshire HP7 9LL (GB); POWELL, Nigel Anthony, GE Healthcare Limited, Amersham, Buckinghamshire HP7 9LL (GB)
(74) Representative: Hammett, Audrey Grace Campbell
(86) International application number: PCT/GB2005/004451
(87) International publication number: WO 2006/054098

(56) References cited:
- WO-A-03/002157
- US-A- 4 889 537
- TIMOTHY J. TEWSON: "Cyclic Sulfur Esters as Substrates for Nucleophilic Substitution. a New Synthesis of 2-Deoxy-2-fluoro-D-glucose" JOURNAL OF ORGANIC CHEMISTRY, vol. 48, 1983, pages 3507-3510, XP002370015
- MIRKO DIKSIC, DEAN JOLLY: "Synthesis of 2-Deoxy-2-fluorohexoses by fluorination of glycals in aqueous media" CARBOHYDRATE RESEARCH, vol. 153, 1986, pages 17-24, XP002370016
- HAMACHER K ET AL: "COMPUTER-AIDED SYNTHESIS (CAS) OF NO-CARRIER-ADDED 2-(18F)FLUORO-2-DEOXY-D-GLUCOSE: AN EFFICIENT AUTOMATED SYSTEM FOR THE AMINOPOLYETHER-SUPPORTED NUCLEOPHILIC FLUORINATION" INTERNATIONAL JOURNAL OF RADIATION APPLICATIONS AND INSTRUMENTATION PART A: APPLIED RADIATION AND ISOTOPES, PERGAMON PRESS LTD., EXETER, GB, vol. 41, no. 1, January 1990 (1990-01), pages 49-53,55, XP000086178

## Description

The present invention relates to a process for the fluoridation of sugar derivatives and in particular, the invention relates to the production of fluoridated glucose. The process is especially useful for the production of radiofluoridated sugar derivatives for use in procedures such as positron emission tomography (PET).

In processes for producing [¹⁸F]-labelled tracer compounds for use in PET, one of the most important factors is the overall non-corrected yield of the synthesis. This is dictated not just by the overall chemical yield of the process, but also by the synthesis time, which is important because of the relatively short half life of [¹⁸F], which is 109.7 minutes.

[¹⁸F]-fluoride ion is typically obtained as an aqueous solution produced by the cyclotron irradiation of an [¹⁸O]-water target. It has been widespread practice to carry out various steps in order to convert [¹⁸F]-fluoride into a reactive nucleophilic reagent such that it is suitable for use in nucleophilic radiolabelling reactions. As with non-radioactive fluoridations, these steps include the elimination of water from the [¹⁸F]-fluoride ion and the provision of a suitable counter-ion (Handbook of Radiopharmaceuticals 2003 Welch & Redvanly eds. ch. 6 pp 195-227). Nucleophilic radiofluoridation reactions are then carried out using anhydrous solvents (Aigbirhio et al, 1995 J. Fluor. Chem. 70 pp 279-87). The removal of water from the fluoride ion is referred to as making "naked" fluoride ion. The presence of significant quantities of water is believed to result in solvation of the fluoride ions, which shields the fluoride from nucleophilic attack on the protected sugar precursor. The removal of water is therefore regarded in the art as a step which is necessary to increase the reactivity of the fluoride as well as to avoid hydroxylated by-products arising from the presence of water (Moughamir et al, 1998 Tett. Letts. 39 pp 7305-6).

US 6,172,207, which relates to a method for synthesising [¹⁸F]-labelled compounds such as [¹⁸F]-fluorodeoxyglucose ([¹⁸F]-FDG), teaches that the fluoridating agent must be made totally anhydrous by additions of acetonitrile to the aqueous solutions followed by azeotropic evaporation to dryness.

The most commonly used process for synthesis of [¹⁸F]-FDG, is that of Hamacher et al, J. Nucl. Med. 27:235-238 (1986) in which the reaction of 1,3,4,6-tetra-O-acetyl-2-0-trifluoromethanesulfonyl-,8-D-mannopyranose with [¹⁸F]fluoride is performed in anhydrous solvent.

There are certain problems which arise from the processes currently used for the production of [¹⁸F]-labelled sugar derivatives; one of these is that removing all of the residual water from the fluoride ion and the solvent takes time and therefore affects the overall non-corrected yield of the synthesis. Also, both the synthetic and mechanical complexity in any automated synthesiser is increased if it is necessary to remove all the residual water. For instance the synthesis may require more drying cycles, whereas a more powerful heater may be required on the synthesiser to effect the synthesis.

Furthermore, it is difficult to ensure that the radiofluoridation reaction is consistently reproducible. This is because there may often be a small amount of residual water in the solvent (for example up to about 1000ppm) and the overall non-corrected yield of the synthesis varies considerably according to the amount of residual water which is present during the labelling reaction. Results have established that it is possible to maintain the water content at 1500 ppm +/- 200 ppm, a deviation of 15%. At 750 ppm such an absolute water variation would have double the deviation in percentage terms.

The present inventors have made the surprising discovery that it is not necessary to carry out the fluoridation of sugar derivatives under anhydrous conditions. Indeed, if the amount of water in the reaction mixture is carefully controlled, the radiochemical purity (and thus the overall yield) of the process is actually improved.

This is particularly surprising in view of the emphasis in the prior art on the necessity of conducting the reaction under anhydrous conditions.

Therefore, in a first aspect of the invention there is provided a process for the preparation of a protected fluoridated glucose derivative, the process comprising reacting a tetraacetyl mannose derivative with a fluoride, characterised in that the reaction is conducted in a solvent containing water in an amount greater than 1000 ppm and less than 50,000 ppm.

The method of the invention has considerable advantages over prior art methods. Firstly, it has been found that far from being decreased, the yield of the reaction is actually increased in the presence of these controlled amounts of water.

Secondly, because the reaction mixture contains water in an amount of greater than 1000ppm, it is much easier to ensure that a consistent amount of water is present in the reaction mixture (for instance by deliberately contaminating the labelling solvent with water) and this means that the reaction conditions are consistently reproducible.

Thirdly, it may be possible to eliminate some of the drying steps used in prior art processes and this would reduce the overall cost of the process in terms of both reagent cost and the manufacturing cost of the synthesiser. It is expected that a simpler process would also positively impact on the overall reliability of the process.

In the present specification, the term "tetraacetyl mannose derivative" refers to a tetraacetyl mannose sugar in which one of the OH groups is replaced by a leaving group and which is optionally bound to a solid support for example as taught in WO-A-03/002157 and the other OH groups of the sugar are protected with an acetyl protecting group.

The term "protected fluoridated glucose derivative" refers to a glucose in which one of the OH groups is replaced by a fluoro and the other OH groups of the sugar are protected with a suitable protecting group.

Suitable protecting groups for the protected sugar derivatives used in the invention are well known in the art and are described, for example, in "Protecting Groups in Organic Synthesis", Theodora W. Greene and Peter G. M. Wuts, published by John Wiley & Sons Inc. The hydroxy groups may be protected by conversion to alkyl esters, for example by reaction with an alkanoyl chloride such as acetyl chloride.

Suitable leaving groups are also well known in the art and include toluene sulfonate and methane sulfonate. It is particularly preferred, however, that the leaving group is a trifluoromethane sulfonate (triflate) group.

The fluoridation reaction will generally be a nucleophilic substitution reaction and replacement of the leaving group by fluoro may cause an inversion of the stereochemistry of the sugar via an SN2 mechanism.

The reaction is especially suitable for the preparation of 2-fluoro-1,3,4,6-tetra-O-acetyl-D-glucose (tetraacetylfluoroglucose or pFDG) from 1,3,4,6-tetra-O-acetyl-2-trifluoromethanesulfonyl-β-D-mannopyranose (tetraacetyl mannose triflate).

Suitable solvents include non protic organic solvents such as acetonitrile, dimethylformamide, dimethylsulfoxide, tetrahydrofuran, dioxan, 1,2-dimethoxyethane, sulfolane or N-methylpyrrolidinone or a mixture of any thereof. However, acetonitrile has been found to be a particularly suitable solvent for the reaction.

Although the improved reaction yield is obtained by including at least 1000 ppm but less than 50,000 ppm water in the solvent, even greater improvements have been achieved when the water content is from about 1000 to 15,000 ppm. The best results were obtained using a solvent with a water content of from about 2000 to 7000 ppm, suitably 2500 to 5000 ppm. In one embodiment, the preferred water content is from 3000ppm to 6000ppm.

As used herein, the term "ppm" , when describing water content of a given solvent, means µgram water/gram.

The correct level of water in the solvent may either be achieved by drying a wet solvent until the desired water content is reached or by adding a suitable amount of water to a dry solvent. The fluoride may be produced in aqueous solution and, in this case, a fluoride solution having the desired water content may be obtained by repeated additions of the solvent followed by evaporation of the solvent/water mix, or by dilution of the aqueous fluoride with the desired organic solvent. Water content of the solvent may also be reduced by using a scavenger resin, such as a functionalised polystyrene resin, for example an epoxide, methylisocyanate, or acid anhydride functionalised resin to remove water from the fluoride solution. Suitable resins are available commercially, for example from Novabiochem. Performance of the scavenger resin may be improved by using a suitable catalyst, for example 4-dimethylaminopyridine (4-DMAP).

In this embodiment, the drying step may be performed by mixing the scavenger resin with the fluoride solution in a container and then separating the scavenger resin by filtration. Alternatively, and particularly suitably when the scavenger resin is used within an automated synthesis apparatus, the scavenger resin may be contained in a vessel through which the fluoride solution is passed. The fluoride solution may be passed through the scavenger resin as a continuous flow, for example at a flow rate of from 0.1 ml/min to 100ml/min, or in batches, so as to permit sufficient residence time on the scavenger resin for the drying to occur.

The reaction may be conducted in solution phase or alternatively, the tetraacetyl mannose derivative may be bound to a solid support to form a resin-linker-vector (RLV) of formula (I):

SOLID SUPPORT-LINKER-tetraacetyl mannose derivative (I)

wherein the solid support is any suitable support;
the tetraacetyl mannose derivative is as defined above;
X is a group which promotes nucleophilic substitution at a specific site on the tetraacetyl mannose derivative, for example, -SO₂O-;
the linker is any suitable organic group which serves to space the reactive site sufficiently from the solid support structure so as to maximise reactivity; for example zero to four aryl groups (for example phenyl) and/or a C₁-C₆ alkyl or haloalkyl (especially fluoroalkyl) chain and optionally one to four additional functional groups such as amide or sulfonamide groups.

RLV systems are discussed at length in WO-A-03/002157, which also gives details of suitable linkers.

The RLV of formula (I) is contacted with a solution of the fluoride, resulting in the displacement of the sugar from the solid support to give a protected fluoridated glucose derivative.

Suitable solid supports are also discussed in WO-A-03/002157 and include polymers such as polystyrene (which may be block grafted, for example with polyethylene glycol), polyacrylamide or polypropylene or glass or silicon coated with such a polymer. Alternatively a resin may be used for example as detailed in WO-A-03/002157. The solid support may be in the form of small discrete particles such as beads or pins or as a coating on the inner surface of a cartridge or on a microfabricated vessel. Carrying out the method of the invention on a solid support enables the product to be obtained in pure form without the need for any additional separation step. This is especially advantageous when the fluoridation is a radiofluoridation as any time saved in the process results in a higher non-corrected radiochemical yield.

The reaction is usually carried out at a temperature of from 5°C to 180°C, but particularly 75°C to 125°C.

The process of the present invention may be carried out as part of an automated synthesis. This is the case whether the reaction takes place in solution or whether the tetraacetyl mannose derivative is bound to a solid phase.

The fluoride which is reacted with the tetraacetyl mannose derivative may be an ionic compound and may be provided with any suitable counter-ion. It is important, however, that the counter ion should be sufficiently soluble in the reaction solvent to maintain the solubility of the fluoride. Therefore, suitable counter ions include large but soft metal ions such as rubidium or cesium, or alternatively non-metallic ions such as tetraalkylammonium and tetraalkylphosphonium. Potassium ions may also be used as counter ions, in which case, in order to increase the reactivity of the fluoride, a phase transfer catalyst such as 4,7,13,16,21,24-hexaoxa-1,10-diazabicyclo-[8,8,8]-hexacosane (sold under the trade mark Kryptofix^{™} 2.2.2) may be added to solubilise the potassium salt in organic solvents.

The process of the present invention is well suited to the production of radiofluoridated derivatives, particularly [¹⁸F]-labelled derivatives and therefore, the fluoride may comprise an [¹⁸F]-fluoride ion.

As briefly discussed above, the [¹⁸F]-fluoride ion may be prepared by the irradiation of an [¹⁸O]-water target and this may be an initial step in the process of the invention.

The process of the present invention is particularly useful for producing radiofluoridated sugar derivatives such as [¹⁸F]-pFDG, which can then be deprotected to give compounds such as [¹⁸F]-FDG, a well-known PET tracer. The deprotection may be an additional step in the process. When the protecting group in the product fluoridated sugar is an ester, for example an acetyl derivative, deprotection may be achieved by acid or base hydrolysis.

Other additional steps include removal of excess [¹⁸F]-fluoride from the solution and removal of the organic solvent. The excess [¹⁸F]-fluoride may be removed by any standard method, for example by ion-exchange chromatography or solid-phase absorbents. Suitable ion exchange resins include BIO-RAD AG 1-X8^{™} and Waters QMA^{™} and suitable solid-phase absorbents include alumina.

The organic solvent may be removed by evaporation at elevated temperature *in vacuo* or by passing a stream of inert gas such as nitrogen or argon over the solution.

The [¹⁸F]-tracer compound which is the final product of these steps may be formulated for administration to a patient, for example as an aqueous solution which may be prepared by dissolving the [¹⁸F]-labelled tracer in sterile isotonic saline which may contain up to 10% of a suitable organic solvent such as ethanol, or alternatively in a suitable buffered solution such as phosphate buffer. Other additives may be used, for example ascorbic acid, which reduces radiolysis.

As already mentioned, a particularly preferred compound which can be prepared by the process of the invention is [¹⁸F]-pFDG and therefore, in a second aspect of the invention there is provided a process for the preparation of [¹⁸F]-pFDG, the process comprising reacting tetraacetyl mannose triflate with [¹⁸F]-fluoride, characterised in that the fluoride is dissolved in a solvent containing water in an amount greater than 1000 ppm and less than 50,000 ppm. In one embodiment of this aspect of the invention, tetraacetyl mannose triflate (1 equivalent) is reacted with [¹⁸F]-fluoride in the presence of Kryptofix^{™} 2.2.2 (0.9 to 1.1 molar equivalent, suitably 0.98 to 0.99 molar equivalent), potassium carbonate ( 0.4 to 0.6 molar equivalent, suitably 0.50 to 0.60 molar equivalent) in acetonitrile containing water in an amount greater than 1000 ppm and less than 50,000 ppm.

Preferred features of the invention are as detailed above for the first aspect. In particular, the process may comprise the initial step of producing the ['8F]-fluoride by irradiating an [¹⁸O]-water target and a further step of converting the [¹⁸F]-pFDG to [¹⁸F]-FDG by acid or alkaline hydrolysis.

The invention will now be described in greater detail with reference to the examples and to the drawings in which:
FIGURE 1 is a plot which shows the correlation of radiochemical purity of a [¹⁸F]-pFDG product with the water content of the solvent.
FIGURE 2 is a graph showing the correlation between the formation of [¹⁸F]-pFDG and pGlucose during the labelling process with resin-linker-vector.
FIGURE 3 is a plot which shows the correlation of radiochemical purity of a [¹⁸F]-pFDG product in an automated synthesis with the water content of the solvent.

### Example 1 - Effect of varying water content on ¹⁸F-labelling of sugars

In this example, three different methods of labelling a sugar with ¹⁸F⁻ were used and the effect of varying the water content of the reaction mixture was assessed.

### a) Resin-linker vector (RLV) Labelling

The ¹⁸F- was introduced to the Tracerlab MX^{™} and dried using the standard drying process used in the production of 2-[18F]fluoro-2-deoxyglucose. Kryptofix^{™} 2.2.2/ potassium carbonate were used to solubilise the fluoride in acetonitrile. On completion of the drying process, followed by dissolution of the fluoride in acetonitrile, a sample of the dried ¹⁸F⁻ in acetonitrile was taken for water content analysis and measured on a Karl Fisher titration meter. Where necessary, extra additions of water were introduced to bring the water content above that normally obtained by the drying process.

A 1 ml Hi Trap^{®} cartridge was packed with around 370 mg of a solid-phase bound protected mannose precursor at a substitution of 0.003 mmol/g. One end of the cartridge was connected via a loop to a syringe driver. The other end of the cartridge was then connected to a N₂ filled vial fitted with a molecular sieve vent. A hot air gun was then used to heat the cartridge at an external cartridge temperature of 80°C.

6x0.5ml of dry acetonitrile was then injected through the system to wash out any impurities and water (naturally present on the resin due for instance from incomplete drying) and the acetonitrile was then discarded. The auto sampler vial containing the dried ¹⁸F⁻ solution (450 µl) was fitted to the apparatus in its place. The syringe driver then moved the dried fluoride backwards and forwards at a flow rate of 180µl/min through 5 cycles. The dried fluoride reacted with the solid-phase mannose precursor to liberate a protected [¹⁸F]-2-deoxy glucose derivative (which post deprotection gives 2-[¹⁸F]-2-deoxyglucose).

A 5µl sample was then taken from the auto sampler vial for thin-layer chromatography (TLC) analysis and spotted onto a silica gel 60 F254 plate and then run in an acetonitrile/water solvent made up to a ratio of 90/10. The radiochemical purity was established on a Perkin Elmer Instant Imager.

### b) Tetra acetyl mannose triflate labelling

A solution of 32mgs of K₂CO₃ dissolved in 600µl of HPLC grade H₂O, plus 150mgs of Kryptofix^{™}2.2.2 dissolved in 2.5ml of acetonitrile was prepared. 0.6 ml of this was added to a glassy carbon reactor together with around 40 MBq of ¹⁸F⁻ in ¹⁸O enriched water. The heater controller was set to 95°C, and the reaction vessel was heated for a total of 35mins to dry the fluoride. The water and acetonitrile were evaporated in a flow of nitrogen.

1 ml of acetonitrile was injected three times in total at 2 minute intervals, to facilitate azeotropic removal of water from the fluoride, the first addition being 20 minutes into the drying process. At 35 minutes the heaterwas turned off and the reaction vessel cooled by compressed air flow external to the reaction vessel to around 45°C.

25mgs of mannose triflate in 2.0ml of CH₃CN was then added to the dried fluoride, followed by mixing. The heater controller was set to 85°C. 2 minutes after reaching the set temperature a sample for TLC analysis was obtained.

The heater was turned off and the reaction vessel cooled by compressed air flow to around 45°C.

The sample for TLC analysis was spotted onto a silica gel strip and then run in an acetonitrile/water solvent made up to a ratio of 95/5. The radiochemical purity was established on a Perkin Elmer Instant Imager. The top of the reaction vessel was then removed and a 50µl sample taken for water content analysis on a Karl Fisher Titration meter.

### c) Automated Labelling

The labelling was performed on a prototype automated synthesis platform comprising 25 three way valves with an onboard heated reactor vessel and a disposable polypropylene cassette. The cassette fluid pathway also allows for SPE purification of intermediates or the final product.

Fluoride was initially trapped on a QMA cartridge and eluted with a solution of 20mg Kryptofix^{™} 2.2.2, 4.1 mg K₂CO₃, 320µL CH₃CN, 80µL H₂O. This was then dried at 105°C/120°C for around 6 minutes in a stream of nitrogen and redissolved in 1.5 ml of around 20 mg/ml tetraacetyl mannose triflate in acetonitrile.

The labelling reaction was performed at a labelling temperature of either 105 or 125°C with reaction times at either 90 or 270 seconds. After labelling the 2[¹⁸F]-2-deoxyglucose was analysed by TLC. The TLC plate was a silica gel 60 F254 employing 95% acetonitrile, 5% water as the developing solvent. The radiochemical purity was established on a Perkin Elmer Instant Imager.

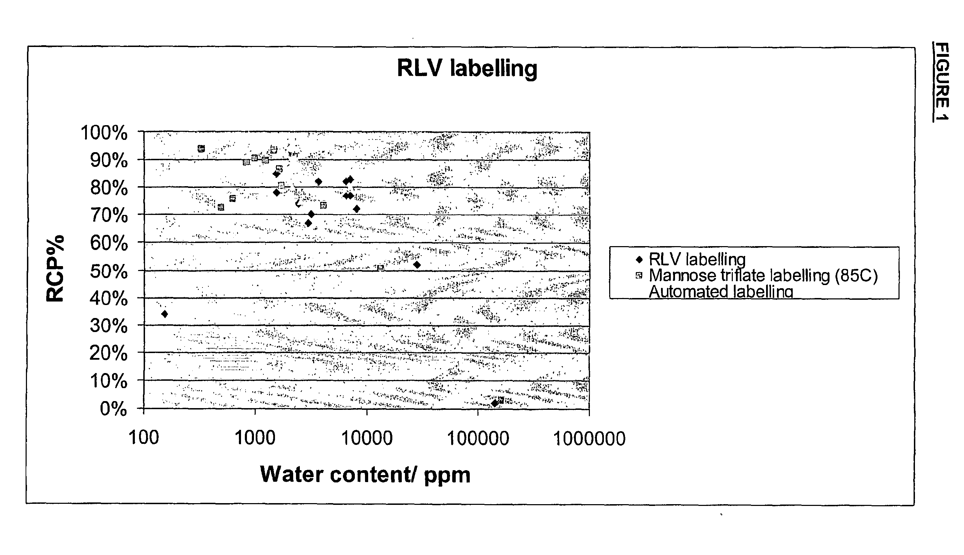

The results of the three experiments of Example 1 are shown in Figure 1, from which it can be seen that the radiochemical purity of the product was relatively low when the water content of the reaction mixture was below 1000ppm but that it improve greatly when the water content was between 1000 and 5000ppm. The plot shows that optimum levels of water in the solvent were between about 2000 and 7000ppm.

### Example 2 - Correlation between the formation of a [¹⁸F]-pFDG and pGlucose during the labelling process.

The 18-fluoride labelling of RLV was achieved in acetonitrile in the presence of Kryptofix^{™} 2.2.2, potassium carbonate and varying quantities of water. Post labelling, the resultant mixture was subject to reversed phase HPLC, running a gradient of 90% solvent A: 10% solvent B (solvent A=0.1 % trifluoroacetic acid solution in water ; solvent B= 0.1 % trifluoroacetic acid solution in acetonitrile) to 5% A, 95% B over 10 minutes at 1 ml/min, and using a Phenomenex Luna 5µm C₁₈ column (4.6mm x 150mm). The integration of the peaks equating to protected glucose at 3 minutes retention time and protected FDG at 6.6 minutes (due primarily to the presence of [19F]-FDG which will be proportional to [18F]-FDG) were determined and correlated.

It is generally believed that the presence of large amounts of water in the reaction mixture results in the formation of large amounts of protected glucose (ratherthan [¹⁸F]-pFDG) as a result of the nucleophilic displacement at the triflate group. Thus it would be expected that a graph plotting concentration of [¹⁸F]-pFDG against concentration of the protected glucose derivative in the product mixture would have a negative slope, with high water content yielding high levels of protected glucose and low levels of [¹⁸F]-pFDG.

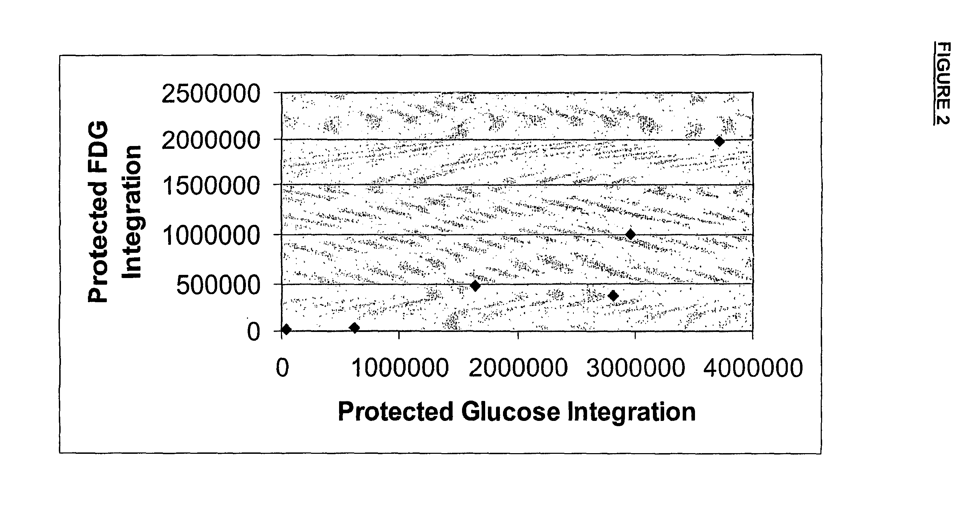

However, from labelling studies on tetraacetyl mannose triflate linked to a resin, it was established that there is a good positive correlation (see Figure 2) between the two peaks on HPLC. This indicates that where a high concentration of water is present, this inhibits the formation of both products.

### Example 3: Automated Synthesis of 1,3,4,6,-tetra-O-acetyl-2-fluoro-β-D-mannopyranose

Sampling the radioactive reaction mixture at the beginning of the labelling procedure was problematic. Water content was therefore measured at the end of the labelling reaction together with the RCP (measured by ITLC). Water content at the beginning of the labelling procedure was then calculated by factoring in the water sequestration as described below.

### Radiolabelling experiment

Synthesis of 1,3,4,6,-tetra-O-acetyl-2-fluoro-β-D-mannopyranose was achieved on an automated synthesiser designed for attachment of a single use disposable cassette. This cassette comprises a 25 valve disposable cassette comprising various reagent-containing vials together with syringes and space for solid-phase extraction cartridges.

A synthesis sequence was executed which trapped around 50 MBq of 18-fluoride in 2 ml water on to a Waters Access PlusQMA cartridge (as its carbonate form) and then eluted the cartridge with a solution of kryptofix and carbonate in acetonitrile/ water (kryptofix 222 - 20.3 mg, potassium carbonate - 4.3 mg, acetonitrile - 320 µl, water - 80 µl) into a heated reactor. This was dried by heating in a stream of dry nitrogen and then a mannose triflate solution in acetonitrile at defined water contents was added to the reactor.

The reaction was allowed to proceed for a further 80 seconds with an external heater temperature of 125°C, then 0.6 ml withdrawn and discarded to waste (to remove any residual water from the lines) and the remainder was transferred to the product vial. The water content of the product vial was determined by Karl Fisher titration using 50 µl of solution and the RCP measured by instant thin-layer chromatography (ITLC). TLC was performed on a silica TLC plates, eluting the spot with 95%acetonitrile, 5% water and then measuring the relative proportion of 18-fluoride and 1,3,4,6,-tetra-O-acetyl-2-fluoro β -D-mannopyranose (in all cases the sole two components) using ITLC.

### Water Sequestration Measurement

Three cold runs were performed, where a defined volume of the reactor liquid was sampled both before and after labelling to see how much the water had dropped through reaction with mannose triflate. This made it possible to factor in the water sequestration into the measured water contents.

A synthesis sequence analogous to the radiolabelling experiment was executed where 2 ml water was passed through a Waters Access PlusQMA cartridge (as its carbonate form) and then the cartridge was eluted with a solution of kryptofix and carbonate in acetonitrile/ water (kryptofix 222 - 20.3 mg, potassium carbonate - 4.3 mg, acetonitrile - 320 µl, water - 80 µl) into a heated reactor. This was dried by heating in a stream of dry nitrogen and then a mannose triflate solution in acetonitrile at defined water contents was added to the reactor.

As soon as the mannose triflate solution had been added to the reactor, 0.6 ml was withdrawn and injected in to a product vial. The labelling reaction was then allowed to proceed for a further 80 seconds at an external heater temperature of 125°C, then the remainder of the solution withdrawn and transferred to a separate product vial. The water content for each vial was measured using a Karl Fisher titrator using 50 µl of solution.

The results of the water sequestration runs are shown in Table 1 which shows the water levels present in the acetonitrile solvent:

**Table 1**

| Pre labelling/ ppm | Post labelling/ ppm |
|---|---|
| 786 | 802 |
| 2603 | 2527 |
| 8433 | 7860 |

At low and medium levels of water there was no significant sequestration of water by reaction with mannose triflate. However, at higher levels of water there was about a 7% drop in water content.

### Radiolabelling Results

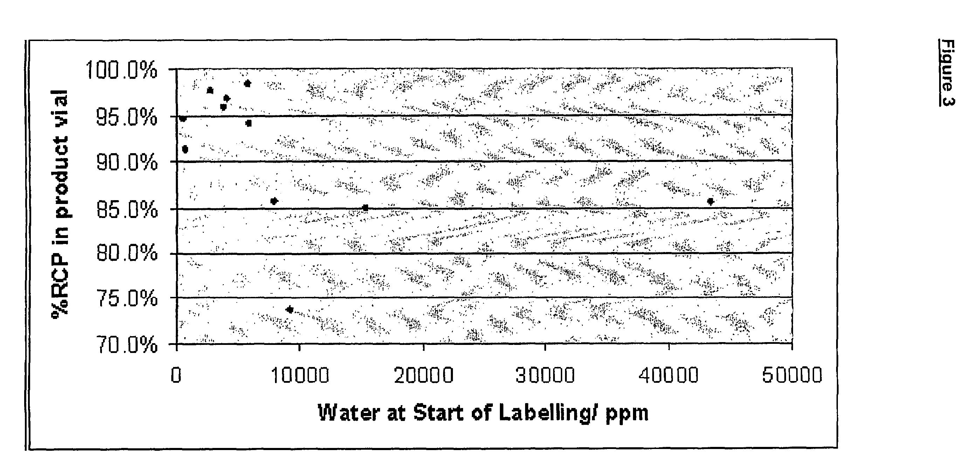

Water content of each radiolabelling reaction was measured and then adjusted by factoring in the water sequestrated by mannose triflate to provide pre-labelling water content. RCP obtained at each water content is given in Table 2 and illustrated in Figure 3.

**Table 2**

| ppm Water | |
|---|---|
| Pre-labelling | RCP% |
| (calculated) | |
| 506 | 94.6% |
| 707 | 91.4% |
| 2803 | 97.6% |
| 3855 | 95.9% |
| 4114 | 96.7% |
| 5779 | 98.4% |
| 5943 | 94.1% |
| 7980 | 85.6% |
| 9206 | 73.6% |
| 15382 | 85.0% |
| 43375 | 85.5% |

These results support a preferred water content of 3000 to 6000ppm. Ignoring the spurious result at 73.6% RCP, the reaction tends to asymptote to around 85% RCP even when the reaction conditions are very wet.

## Claims

1. A process for the preparation of a protected fluoridated glucose derivative, the process comprising reacting a tetraacetyl mannose derivative with a fluoride, **characterised in that** the reaction is conducted in a solvent containing water in an amount greater than 1000 ppm and less than 50,000 ppm.

2. A process as claimed in claim 1 for the preparation of 2-fluoro-1,3,4,6-tetra-O-acetyl-D-glucose (tetraacetylfluoroglucose or pFDG) from 1,3,4,6-tetra-O-acetyl-2-trifluoromethanesulfonyl-β-D-mannopyranose (tetraacetyl mannose triflate).

3. A process as claimed in claim 1 or 2, wherein the solvent is selected from acetonitrile, dimethylformamide, dimethylsulfoxide, tetrahydrofuran, dioxan, 1,2-dimethoxyethane, sulfolane and N-methylpyrrolidinone.

4. A process as claimed in claim 3 wherein the solvent is acetonitrile.

5. A process as claimed in any one of claims 1 to 4, wherein the water content of the solvent is from 1000 to 15,000 ppm.

6. A process as claimed in claim 5, wherein the water content of the solvent is from 2000 to 7000 ppm.

7. A process as claimed in claim 5 wherein the water content of the solvent is 3000ppm to 6000ppm.

8. A process as claimed in any one of claims 1 to 7 which is conducted in solution phase.

9. A process as claimed in any one of claims 1 to 8 which is automated.

10. A process as claimed in any one of claims 1 to 9, wherein the fluoride is an ionic fluoride with a potassium counter ion and a phase transfer catalyst such as 4,7,13,16,21,24-hexaoxa-1,10-diazabicyclo-[8,8,8]-hexacosane is added to the fluoride.

11. A process as claimed in any one of claims 1 to 10 for the preparation of a radiofluoridated sugar derivative.

12. A process as claimed in claim 11, wherein the radiofluoridated sugar derivative is an [¹⁸F]-labelled sugar derivative.

13. A process as claimed in any one of claims 1 to 12 for the preparation of [¹⁸F]-pFDG, the process comprising reacting tetraacetyl mannose triflate with [¹⁸F]-fluoride.

14. A process as claimed in any one of claims 1 to 13 further comprising, in any order, one or more additional step of:
i. removal of excess fluoride from the solution;
ii. deprotecting the protected fluoridated sugar derivative to give a deprotected fluoridated sugar derivative;
iii. removal of the organic solvent; and
iv. formulating the deprotected fluoridated sugar derivative in aqueous solution.

## Patentansprüche

1. Verfahren zur Herstellung eines geschützten fluorierten Glucosederivats, wobei das Verfahren das Umsetzen eines Tetraacetylmannosederivats mit einem Fluorid umfasst, **dadurch gekennzeichnet, dass** die Reaktion in einem Lösungsmittel durchgeführt wird, welches Wasser in einer Menge über 1000 ppm und unter 50000 ppm enthält.

2. Verfahren nach Anspruch 1 zur Herstellung von 2-Fluor-1,3,4,6-tetra-O-acetyl-D-glucose (Tetraacetylfluorglucose oder pFDG) aus 1,3,4,6-Tetra-O-acetyl-2-trifluormethansulfonyl-β-D-mannopyranose (Tetraacetylmannosetriflat).

3. Verfahren nach Anspruch 1 oder 2, wobei das Lösungsmittel gewählt wird aus Acetonitril, Dimethylformamid, Dimethylsulfoxid, Tetrahydrofuran, Dioxan, 1,2-Dimethoxyethan, Sulfolan und N-Methylpyrrolidinon.

4. Verfahren nach Anspruch 3, wobei das Lösungsmittel Acetonitril ist.

5. Verfahren nach einem der Ansprüche 1 bis 4, wobei der Wassergehalt des Lösungsmittels 1000 bis 15000 ppm beträgt.

6. Verfahren nach Anspruch 5, wobei der Wassergehalt des Lösungsmittels 2000 bis 7000 ppm beträgt.

7. Verfahren nach Anspruch 5, wobei der Wassergehalt des Lösungsmittels 3000 ppm bis 6000 ppm beträgt.

8. Verfahren nach einem der Ansprüche 1 bis 7, welches in Lösungsphase durchgeführt wird.

9. Verfahren nach einem der Ansprüche 1 bis 8, welches automatisiert ist.

10. Verfahren nach einem der Ansprüche 1 bis 9, wobei das Fluorid ionisches Fluorid mit einem Kaliumgegenion ist und ein Phasentransferkatalysator wie 4,7,13,16,21,24-Hexaoxa-1,10-diazabicylco-[8,8,8]-hexacosan zum Fluorid gegeben wird.

11. Verfahren nach einem der Ansprüche 1 bis 10 zur Herstellung eines radiofluorierten Zuckerderivats.

12. Verfahren nach Anspruch 11, wobei das radiofluorierte Zuckerderivat ein [¹⁸F]-markiertes Zuckerderivat ist.

13. Verfahren nach einem der Ansprüche 1 bis 12 zur Herstellung von [¹⁸F]-pFDG, wobei das Verfahren das Umsetzen von Tetraacetylmannosetriflat mit [¹⁸F]-Fluorid umfasst.

14. Verfahren nach einem der Ansprüche 1 bis 13, welches weiterhin, in beliebiger Reihenfolge, einen oder mehrere zusätzliche Schritte umfasst:
i. Entfernung überschüssigen Fluorids aus der Lösung;
ii. Entschützung des geschützten fluorierten Zuckerderivats, damit sich ein entschütztes fluoriertes Zuckerderivat ergibt;
iii. Entfernung des organischen Lösungsmittels; und
iv. Formulierung des entschützten fluorierten Zuckerderivats in wässriger Lösung.

## Revendications

1. Procédé de préparation d'un dérivé de glucose fluoré protégé, le procédé comprenant la mise en réaction d'un dérivé de mannose tétra-acétylique avec un fluorure, **caractérisé en ce que** la réaction est mise en oeuvre dans un solvant contenant de l'eau en une quantité supérieure à 1000 ppm et inférieure à 50 000 ppm.

2. Procédé selon la revendication 1, destiné à assurer la préparation de 2-fluoro-1,3,4,6-tétra-O-acétyle-D-glucose (tétra-acétylfluoroglucose ou pFDG) à partir de 1,3,4,6-tétra-O-acétyle-2-trifluorométhanesulfonyl-p-D-mannopyranose (triflate de mannose tétra-acétylique).

3. Procédé selon la revendication 1 ou 2, dans lequel le solvant est sélectionné à partir d'acétonitrile, de diméthylformamide, de diméthylsulfoxyde, de tétrahydrofurane, de dioxane, de 1,2-diméthoxyéthane, de sulfolane et de N-méthylpyrrolidinone.

4. Procédé selon la revendication 3, dans lequel le solvant est de l'acétonitrile.

5. Procédé selon l'une quelconque des revendications 1 à 4, dans lequel la teneur en eau du solvant est comprise entre 1000 et 15 000 ppm.

6. Procédé selon la revendication 5, dans lequel la teneur en eau du solvant est comprise entre 2000 et 7000 ppm.

7. Procédé selon la revendication 5, dans lequel la teneur en eau du solvant est comprise entre 3000 ppm et 6000 ppm.

8. Procédé selon l'une quelconque des revendications 1 à 7 qui est mis en oeuvre en phase de solution.

9. Procédé selon l'une quelconque des revendications 1 à 8 qui est automatisé.

10. Procédé selon l'une quelconque des revendications 1 à 9, dans lequel le fluorure est un fluorure ionique avec un contre-ion potassium, et un catalyseur de transfert de phase, tel que le 4,7,13,16,21,24-hexa-oxa-1,10-diazabicyclo-[8,8,8]-hexacosane, est ajouté au fluorure.

11. Procédé selon l'une quelconque des revendications 1 à 10 destiné à assurer la préparation d'un dérivé de sucre radiofluoré,

12. Procédé selon la revendication 11, dans lequel le dérivé de sucre radiofluoré est un dérivé de sucre marqué par [18F].

13. Procédé selon l'une quelconque des revendications 1 à 12 destiné à assurer la préparation de [18F]-pFDG, le procédé comprenant la mise en réaction de triflate de mannose tétra-acétylique avec un fluorure marqué par [18F].

14. Procédé selon l'une quelconque des revendications 1 à 13 comprenant, en outre, suivant un ordre quelconque, une ou plusieurs étapes supplémentaires consistant en :
i. l'élimination de l'excès de fluorure de la solution.
ii. la déprotection du dérivé de sucre fluoré protégé afin d'obtenir un dérivé de sucre fluoré déprotégé ;
iii. l'élimination du solvant organique; et
iv. la formulation du dérivé de sucre fluoré déprotégé en solution aqueuse.
